Europäisches Patentamt

European Patent Office

Office européen des brevets

(19)

(11) Veröffentlichungsnummer: **0 030 610**
**B1**

(12) # EUROPÄISCHE PATENTSCHRIFT

(45) Veröffentlichungstag der Patentschrift: **05.03.86**

(21) Anmeldenummer: **80106584.8**

(22) Anmeldetag: **27.10.80**

(51) Int. Cl.⁴: **A 61 B 5/00**, G 01 N 21/21, G 01 N 33/66

(54) Verfahren und Vorrichtung zur quantitativen Bestimmung optisch aktiver Substanzen.

(30) Priorität: **31.10.79 DE 2944113**

(43) Veröffentlichungstag der Anmeldung:
**24.06.81 Patentblatt 81/25**

(45) Bekanntmachung des Hinweises auf die Patenterteilung:
**05.03.86 Patentblatt 86/10**

(84) Benannte Vertragsstaaten:
**AT CH FR GB LI NL**

(56) Entgegenhaltungen:
**FR-A-2 393 296**

(73) Patentinhaber: **Müller, Arno, Dr. Dipl.-Phys.
Gartenhalde 22
D-7900 Ulm-Mähringen (DE)**

(72) Erfinder: **Müller, Arno, Dr. Dipl.-Phys.
Gartenhalde 22
D-7900 Ulm-Mähringen (DE)**

(74) Vertreter: **Beetz, sen., Richard, Dipl.-Ing.
Patentanwälte Dipl.-Ing. R. Beetz sen. Dipl.-Ing.
K. Lamprecht,Dr.Ing. R. Beetz jr. et al
Rechtsanwalt Dipl.-Phys. Dr. jur. U. Heidrich,
Dr.-Ing. W. Timpe Dipl.-Ing. J. Siegfried Priv.-
Doz. Dipl.-Chem. Dr.rer.nat. W. Schmitt-Fumian
Steinsdorfstrasse 10 D-8000 München 22 (DE)**

Courier Press, Leamington Spa, England.

EP 0 030 610 B1

**Beschreibung**

Die Erfindung betrifft ein hochempfindliches Verfahren zur quantitativen Bestimmung optisch aktiver Substanzen auch in sehr kleinen Konzentrationen in wäßrigen oder nichtwäßrigen Lösungen, durch Polarimetrie sowie ein hochempfindliches Polarimeter, das sich zur Durchführung dieses Verfahrens eignet und sowohl in vivo, dh direkt am oder im lebenden Organismus, als auch in vitro eingesetzt werden kann, beispielsweise zur Bestimmung von Glucose oder Fructose im Blut, Plasma oder Serum.

Bei der Bestimmung der Konzentration optisch aktiver Substanzen kommt der Ermittlung der Glucosekonzentration im Hinblick auf die Erkennung, Behandlung und Überwachung von Diabetes besondere Bedeutung zu.

Es ist bereits bekannt, daß die Glucosekonzentration, meist im Serum, entweder enzymatisch oder zB durch visuelle Kolorimetre, Titrimetrie oder Photometrie bestimmt werden kann (Pikrinsäure - Methode, Glucoseoxidase-, Peroxidase-, Hexokinase-Methode). Aus den DE-OSen 2 200 119 und 2 326 265 ist ferner bekannt, daß eine Brennstoffzelle in einen lebenden Organismus eingepflanzt werden kann, die über eine telemetrische Vorrichtung einige charakteristische Meßwerte (zB den pH-Wert, die Glucosekonzentration ua) in elektrische Signale umsetzt. Es ist auch bereits vorgeschlagen worden, die Blutglucosekonzentration durch Messung der Absorption eines Lichtstrahls im Blut bei einer bestimmten Wellenlänge zu bestimmen (vgl die US—PS 3 958 560; N. Kaiser, Optics Communication *11*, Nr. 2 (1974) 175). Derartigen Messungen stehen jedoch wegen der Vielzahl von Absorptionsbanden organischer Moleküle grundsätzliche Schwierigkeiten entgegen.

Aus der DE—OS 2 724 543 ist bekannt, die Glucosekonzentration polarimetrisch in vitro oder in vivo zu messen. Da aber bei der polarimetrischen Bestimmung zB der Glucose im Blut durch die Anwesenheit anderer optisch aktiver Substanzen eine Untergrunddrehung auftritt, die sich dem glucosebedingten Drehwert überlagert, erlaubt dieses Verfahren nur Relativmessungen. Zudem ist der apparative Aufwand infolge der erforderlichen hohen Meßempfindlichkeit von $10^{-4}$ bis $10^{-6\circ}$ relativ hoch.

In der FR—OS 2 393 296 sind ferner ein Verfahren und eine Vorrichtung zur quantitativen Bestimmung optisch aktiver Substanzen, deren Konzentration einer zeitlichen Änderung unterliegt, angegeben, bei denen zur Absolutbestimmung der Konzentration einer optisch aktiven Substanz in Gegenwart anderer, ebenfalls optisch aktiver Substanzen mit davon hinreichend verschiedener zeitlicher Konzentrationsänderung die Signalkomponente mit der Konzentrationsänderungsfrequenz der zu bestimmenden optisch aktiven Substanz von den übrigen Signalkomponenten abgetrennt wird. Das aus dieser Druckschrift bekannte Polarimeter weist jedoch ebenso wie sämtliche herkömmlichen Polarimeter einen diskreten Analysator und damit begrenzte Empfindlichkeit auf, die für diagnostische Zwecke, insbesondere zur Glucosebestimmung, nicht ausreichend ist.

Der Erfindung liegt die Aufgabe zugrunde, ein hochempfindliches schnelles und reproduzierbares Verfahren sowie ein hochempfindliches Polarimeter zur quantitativen Bestimmung optisch aktiver Substanzen auch in sehr kleinen Konzentrationen wäßrigen oder nichtwäßrigen und insbesondere biochemischen und biologischen Systemen anzugeben, das sich zur Durchführung dieses Verfahrens eignet und ferner auch zur direkten in-vivo-Messung des Blutglucosespiegels, beispielsweise bei Diabetikern, etwa auf transkutanem bzw verletzungsfreiem Wege eingesetzt werden kann.

Das erfindungsgemäße Polarimeter soll dabei auf einem einfachen apparativen Konzept beruhen und eine höhere Empfindlichkeit als herkömmliche Polarimeter aufweisen. Es soll sich ferner nicht nur stationär einsetzen lassen, sondern auch aufgrund seines Konzepts hinreichend klein und leicht ausführbar sein, so daß es beispielsweise am Körper getragen oder teilweise oder ganz in ihn implantiert werden kann.

Die Aufgabe wird anspruchsgemäß gelöst.

In der Unteransprüchen sind vorteilhafte Ausführungsformer angegeben.

Das erfindungsgemäße Verfahren zur Bestimmung gelöster optisch aktiver Substanzen in wäßrigen oder nichtwäßrigen Lösungen durch Polarimetrie unter

— Bestrahlung der Probe mit linear polarisiertem Licht,
— Strahlteilung des Lichtstrahls nach dem Durchlaufen der Probe mit einem Strahlteiler und Erzeugung eines Meßstrahls und eines Referenzstrahls,
— Bildung eines Differenz- oder Quatientensignals aus den mit entsprechenden Detektoren erfaßten Signalen des Meß- und Referenzstrahls und Endverstärkung des resultierenden Signals zur Anziege oder Registrierung

ist dadurch gekennzeichnet, daß zur gleichzeitigen Strahlteilung und Erzielung eine Analysatorwirkung der Lichtstrahl nach der Probe unter einem Einfallswinkel (α) auf eine optische Grenzfläche gerichtet wird, der zwischen dem Polarisationswinkel und dem Grenzwinkel der Totalreflexion liegt.

In Fällen, in denen die Konzentration einer gelösten optisch aktiven Substanz in Gegenwart anderer optisch aktiver Substanzen bestimmt werden soll, deren zeitliche Änderung von der zeitlichen Änderung der anderen optisch aktiven Substanz hinreichend verschieden ist, wird in an sich bekannter Weise die Signalkomponente mit der Konzentrationsänderungsfrequenz der zu bestimmenden optisch aktiven Substanz durch Signaltrennung von den Übrigen Signalkomponenten abgetrennt. Diese Verfahren ist auch bei nichtperiodischen bzw nicht wiederholten Konzentrationsänderungen, zB bei abklingender Konzentration der zu bestimmenden Substanz, anwendbar.

Diese Signalteilung kann vorteilhaft durch Frequenzbandtrennung vorgenommen werden, wobei hierzu günstig ein Hoch- bzw Tiefpaß bzw ein Bandpaß herangezogen werden können. Ebenso vorteilhaft kann die Signaltrennung auch durch Differentiation und nachfolgende Integration bzw durch Differentiation, Anwendung eines Hoch- bzw Tiefpasses und nachfolgende Integration vorgenommen werden. Eine etwa vorhandene Untergrunddrehung kann ferner durch Addition einer Gleichstromkomponente kompensiert werden. Darüber hinaus können vor oder im Endverstärker evtl bei der Frequenzbandtrennung auftretende Amplituden- und/oder Phasenfehler ausgeglichen werden.

Die oblige Signaltrennung ist auf alle Systeme anwendbar, bei denen die zeitliche Konzentrationsänderung der optisch aktiven Substanzen und damit die zeitliche Änderung der entsprechenden Inkremente des optischen Drehwerts hinreichend voneinander verschieden sind. Die Trennbarkeit der zu den einzelnen optisch aktiven Substanzen gehörigen Drehwertkomponenten hängt von der Steilheit der Dämpfungskurve des zur Frequenzbandtrennung verwendeten Bandpasses ab. Mit üblichen RC-Gliedern lassen sich beispielsweise Frequenzen, die um den Faktor 1,5 voneinander verschieden sind, noch ohne weiteres voneinander trennen. Dieser Faktor stellt jedoch keinen Grenzwert dar.

Die Meßdauer hängt von der Konzentrationsänderungsfrequenz der zu bestimmenden optisch aktiven Komponente ab. Bei einer mittleren Frequenz von etwa $10^{-4}$ Hz (die Glucosekonzentration im Blut ändert sich zB mit einer maximalen Frequenz von etwa $8 \times 10^{-3}$ Hz, während sich die Konzentration der Proteine und Blutfette im Normalfall mit einer maximalen Frequenz von etwa $10^{-5}$ Hz ändert) resultiert eine Periodendauer von $10^4$ s und damit eine Mindest-Meßdauer von etwa 3 h.

Das erfindungsgemäße hochempfindliche Polarimeter weist eine Lichtquelle für polarisiertes Licht, einen Probenteil zur Aufnahme der Probe, einen Strahlteiler, je einen Detektor für den Meß- sowie den Referenzstrahl, einen Differenzverstärker oder Quotientenbildner, einen Signalverstärker sowie eine Anzeige-, Ausgabe- oder Registriereinrichtung auf und ist dadurch gekennzeichnet, daß als Strahlteiler sowie als Analysator

(A) eine planparallele Platte derart angeordnet ist, daß der zum Einfallslot auf der reflektierenden Fläche hin gemessene Einfallswinkel α zwischen dem Grenzwinkel der Totalreflexion und dem Polarisationswinkel liegt, oder

(B) ein Prisma mit den Prismenwinkeln α, 2α und 180—3α im Dreiecksquerschnitt derart angeordnet ist, daß der zum Einfallslot auf der reflektierenden Fläche hin gemessene Einfallswinkel α gleich dem Prismenwinkel α ist, wobei α zwischen

dem Grenzwinkel der Totalreflexion und dem Polarisationswinkel liegt.

Die quantitative Bestimmung optisch aktiver Substanzen in sehr kleinen Konzentrationen setzt eine hohe Empfindlichkeit der Bestimmungsmethode bzw der entsprechenden Polarimeters voraus. Bei herkömmlichen Polarimetern erfolgt die Ermittlung des Drehwerts des polarisierten Lichts nach Durchlaufen der Probe mit einem diskreten Analysator, der eine Änderung der Intensität des in seiner Polarisationsebene gedrehten Lichts bewirkt (vgl die DE—OS 2 724 543), da er nur für Licht voll durchlässig ist, das in seiner Polarisationsrichtung schwingt. Ist die Polarisationsebene des auffallenden Lichts um den Winkel φ zur Polarisationsrichtung des Analysators gedreht, so ergibt sich, da die Intensität proportional zum Quadrat der Amplitude ist, der Zusammenhang

$$I = I_o \cdot \cos^2 \varphi \qquad (1).$$

Für die Empfindlichkeit derartiger herkömmlicher Polarimeter folgt daraus

$$\frac{dI}{d\varphi} = I_o \cdot 2 \cdot \cos \varphi \cdot \sin \varphi \qquad (2).$$

Gleichung (2) stellt den formelmäßigen Ausdruck für die bekannte Tatsache dar, daß bei einer Verdrehung der Polarisationsebene um 45° zur ursprünglichen Polarisationsrichtung maximale Empfindlichkeit resultiert. Bei Anordnungen mit Strahlteiler, wie etwa aus der CH—PS 441 814 sowie der DE—OS 2 724 543 bekannt, ist die Empfindlichkeit von der Absolutintensität unabhängig, und es gilt

$$\frac{dI}{d\varphi} = 1 \qquad (3).$$

Die Empfindlichkeit herkömmlicher Polarimeter, bei denen die Drehung der Polarisationsebene des nach der Probe resultierenden Lichts gegenüber der Polarisationsebene des Lichts vor der Probe mit einem Analysator bestimmt wird, ist daher theoretisch begrenzt.

Eine Erhöhung der Empfindlichkeit wird zum einen dadurch erreicht, daß das polarisierte Licht der Lichtquelle, bevor es zur Probe gelangt, zunächst einen Modulator durchläuft. Der Modulator, zB ein Faraday-Modulator, arbeitet im Nieder- oder Hochfrequenzbereich, je nachdem, welche höchste Frequenz die Lichtdetektoren verarbeiten können. Als Beispiel sei im einfachsten Fall 1000 Hz genannt. Anstelle der Phasenmodulation kann der Lichtstrahl ebenso auch frequenz- oder amplitudenmoduliert werden, wobei dann die Demodulationseinrichtung auf die jeweilige Modulationsart abgestimmt ist. Die Erzielung einer Analysatorwirkung erfolgt erfindungsgemäß durch Ausnutzung der Abhängigkeit des Reflexionskoeffizienten vom Azimutwinkel.

Das dabei erhaltene nieder- oder hochfrequente Signal kann dann in einem Wechselstromverstärker (einem selektiven Verstärker oder Lock-in-Verstärker) verstärkt und dann demoduliert werden. Auf diese Weise kann das die Empfindlichkeit begrenzende Signal-Rausch-Verhältnis erfindungsgemäß um mindestens eine Größenordnung verbessert werden.

Die erfindungsgemäß erzielte Empfindlichkeitssteigerung beruht zum anderen darauf, daß der bisher als zwingend erforderlich angesehene diskrete Analysator, mit dem die Probenbedingte Drehung der Polarisationsebene des eingestrahlten Lichts ermittelt wurde, weggelassen wird:

Trifft ein polarisierte Lichtstrahl, der ggf zuerst moduliert wurde, nach Durchlaufen der Probe auf eine planparallel Platte, die gleichzeitig auch als Strahlteiler dient, so ist die reflektierte Intensität vom Einfallswinkel α sowie vom Azimutwinkel φ, dh dem Winkel der Schwingungsebene des eingestrahlten Lichts zur Einfallsebene, abhängig; der durch die Platte hindurchgehende Anteil, dh die Intensität des gebrochenen Lichts, ist ebenfalls in bekannter Weise vom Winkel α abhängig (vgl zB Bergmann, Schäfer, Lehrbuch der Experimentalphysik, Bd. III, Optik, Walter de Gruyter & Co.Verlag, Berlin (1966)). Dabei ist bemerkenswert, daß der Reflexionskoeffizient im Bereich oberhalb des Polarisationswinkels stark zunimmt, während der Durchlässigkeitskoeffizient im genannten Bereich abnimmt.

Die Empfindlichkeit kann aus der Änderung des Reflexionskoeffizienten sowie des Durchlässigkeitskoeffizienten durch Bildung der Ableitung nach dem Winkel α berechnet werden. In erster Näherung genügt es, den Reflexionskoeffizienten zu betrachten, da sich der Durchlässigkeitskoeffizient weniger stark mit α ändert. Weiterhin muß noch die Ableitung nach dem Azimutwinkel φ durchgeführt werden, da bei der polarimetrischen Messung ja nur dieser geändert wird. Daraus folgt für die Änderung des Reflexionskoeffizienten $\rho_p$ als Empfindlichkeit des erfindungsgemäßen Polarimeters:

$$\frac{d\rho_p}{d\varphi_e} = \frac{\partial\rho_p}{\partial\alpha}\cdot\frac{\partial\alpha}{\partial\varphi_e} = \frac{n^3(n^2-1)\cdot\left\{\left(\cos\alpha\sqrt{1-\dfrac{\sin^2\mu}{n^2}}+\dfrac{\sin^2\alpha}{n}\right)^2+tg^2\varphi_r\left(\cos\alpha\sqrt{1-\dfrac{\sin^2\alpha}{n^2}}-\dfrac{\sin^2\alpha}{n}\right)^2\right\}}{tg\varphi_r\sqrt{n^2-\sin^2\alpha}\{n^2-\sin^2\alpha+2n^2\cos\alpha\sqrt{n^2-\sin^2\alpha}+n^4\cos^2\alpha\}[2-(1+\dfrac{1}{n})\cdot2)\sin^2\alpha]} \quad (4),$$

wobei bedeuten:

$\rho_p$ den Reflexionskoeffizienten (Schwingungsebene der Strahlung parallel zur Einfallsebene),
α den Einfallswinkel der Strahlung, zum Einfallslot hin gemessen,
$\varphi_e$ den Winkel der einfallenden Strahlung zur Einfallsebene,
$\varphi_r$ den Winkel der reflektierten Strahlung zur Einfallsebene und
n den Bechungsindex (Luft-reflektierendes Medium).

Die quantitative Auswertung der Gleichung (4) für die Werte n=1,5, $\varphi_e$=5,7° und α=70° ergibt unter Mitberücksichtigung der Änderung des Durchlässigkeitskoeffizienten den Faktor 16 für die Steigerung der Empfindlichkeit gegenüber einem herkömmlichen Polarimeter, für das Gleichung (3) gilt; der Faktor 16 setzt sich aus dem Faktor 8 der Änderung des Reflexionskoeffizienten und dem Faktor 2 der Änderung des Durchlässigkeitskoeffizienten zusammen. Ein derartiges erfindungsgemäßes Polarimeter mit einer planparallelen Platte als Strahlteiler und Analysator weist somit eine gegenüber herkömmlichen Polarimetern wesentlich erhöhte Empfindlichkeit auf.

Daraus folgt, daß erfindungsgemäß, der herkömmliche Analysator entfallen kann, wenn die Reflexion am optisch dünneren oder dichteren Medium zur Erzielung einer Analysatorwirkung ausgenutzt wird; dabei kann der reflektierte Strahl als Meßstrahl und der gebrochene Strahl als Referenzstrahl verwendet werden, wobei diese Zuordnung jedoch nicht zwingend ist. Anstelle der planparallelen Platte, die bei der erfindungsgemäßen Anordnung als Strahlteiler sowie als Analysator verwendet wird, läßt sich gemäß der Erfindung auch ein Prisma verwenden. Dabei wird das Prisma so durchstrahlt, daß der polarisierte Lichtstrahl, der von der Probe kommt, senkrecht auf eine Prismenfläche auftrifft, so daß keine Reflexion auftritt. Erst an der rückwärtigen Prismenfläche zum dünneren Medium hin wird der Lichtstrahl in einen reflektierten und in einen gebrochenen Strahl aufgeteilt. Der reflektierte Strahl wird beim Austritt aus dem Prisma ebenfalls gebrochen. Somit resultiert eine im Prinzip gleiche Anordnung wie bei der planparalellen Platten erläutert; der reflektierte Strahl liefert vorzugsweise die Signalspannung, während der gebrochene Strahl die Referenzspannung ergibt.

Diese Anordnung mit einem Prisma hat den Vorteil, daß sich der Reflexionskoeffizient bei geeigneter Wahl der Prismenwinkel sehr start mit dem Winkel α ändert. Daraus resultiert eine weiter verbesserte Empfindlichkeit. Darüber hinaus ist bei dieser Anordnung eine Änderung der Intensität des gebrochenen Lichts, dh die Änderung des Durchlässigkeitskoeffizienten, ohne Einfluß, da man die Prismenwinkel so wählen kann, daß der Referenzstrahl und der Signalstrahl das Prisma unter dem gleichen Winkel verlassen. Dies ist zB bei einem Prisma mit den α, 2α und (180—3α) der Fall. Die theoretisch für diesen Fall erreichbare Empfindlichkeit kann ebenso wie Gleichung (4) aus den Fresnelschen Formeln hergeleitet werden. Dabei resultiert ein prinzipiell gleicher Ausdruck mit dem einzigen Unterschied, daß gegenüber Gleichung (4) anstelle des Brechungsindex n sein Reziprokwert 1/n steht. Obwohl die so erhaltene Gleichung formal Gleichung (4) entspricht, führt sie doch su erheblich unterschiedlichen Konsequenzen. So wird zB die Empfindlichkeit für den Grenzwinkel der Totalreflexion unendlich groß. Sie ist jedoch bereits für den Polarisationswinkel je nach dem Azimutwinkel φ um den Faktor 3 größer als jene bei Verwendung einer planparallelen Platte. Insgesamt ergibt sich bei dieser Anordnung eine gegenüber herkömmlichen Polarimetern um zwei Größenordnungen höhere Empfindlichkeit. Die ausnutzbare Empfindlichkeit des erfindungsgemäßen Polarimeters ist vom geforderten Meßbereich abhängig, beispielsweise von der maximal zu messenden Winkeländerung. Wenn beispielsweise ein Meßbereich von ±0,5° angenommen wird, ergibt sich rechnerisch mit n=1,5, $\varphi_e$=5,7° und α=41,2° eine um den Faktor 100 verbesserte Empfindlichkeit.

Die Erfindung wird im folgenden anhand der Zeichnung näher erläutert; es zeigen:

Fig. 1 bis 4: Schematische Darstellungen verschiedener herkömmlicher Polarimeter,

Fig. 5 bis 8: schematische Darstellungen des erfindungsgemäßen Polarimeters und

Fig. 9: eine spezielle Bauweise des erfindungsgemäßen Polarimeters zur verletzungsfreien transkutanen Blutzuckerbestimmung.

In den Figuren besitzen die Bezugszahlen folgende Bedeutung:

1=Lichtquelle, die linear polarisiertes Licht emittiert,
2=Modulator,
3=Probe,
4=λ/4-Plättchen,
4a, 4b=Polarisationsfilter,
5=Strahlteiler und Analysator,
6=Analysator,
7 und 7*=Detektor, beispielsweise Photodiode, Photomultiplier, Phototransistor udgl,
8=Differenzverstärker oder Quotientenbildner,
9=Verstärker, Demodulator,
10=Anzeige-, Ausgabe- oder Registriereinrichtung,
11=Frequenzgenerator,
12=Differenzierglied,
13=Signalteiler,
14=Integrator oder Gleichstromaddierer,
15=Verstärker,

* bezeichnet das Referenzsignal.

+bzw—bedeuten bei einem Differenzverstärker den positiven bzw negativen Eingang, bei einem Quotientenbildner den Zähler bzw Nenner,

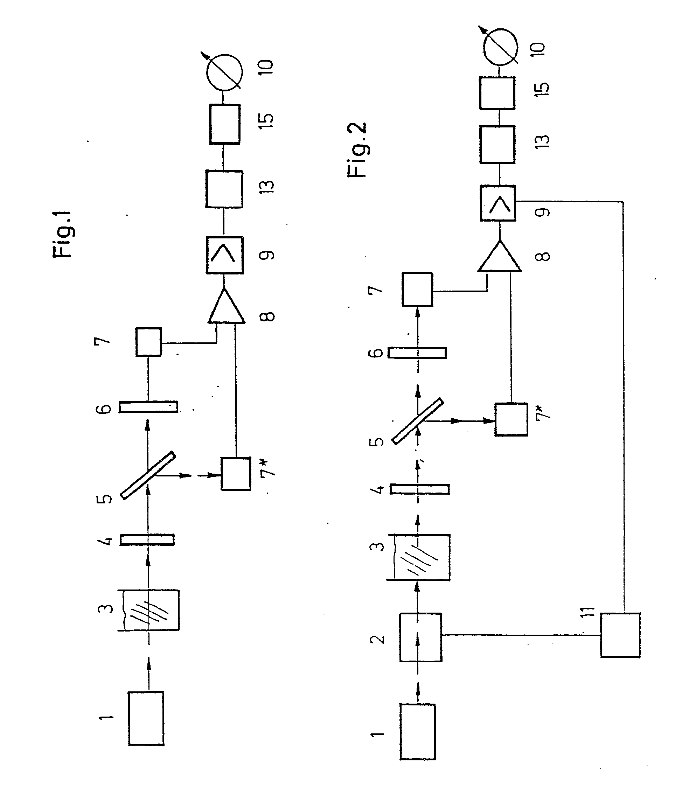

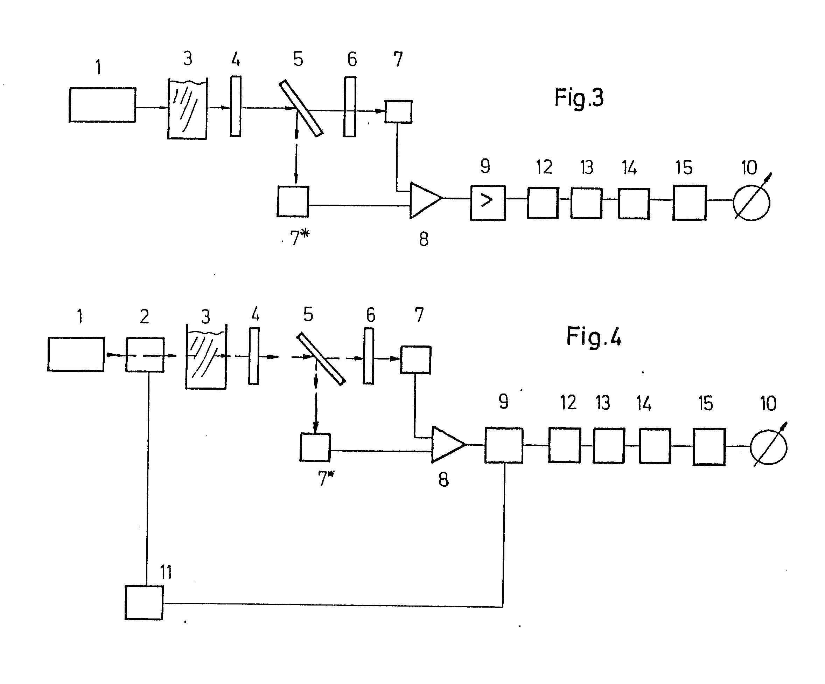

In Fig. 1 ist ein herkömmliches Polarimeter dargestellt, bei dem die Selektion des der zu bestimmenden Komponente zugeordneten Drehwerts durch einen Signalteiler 13 erfolgt, beispielsweise einen Hochpaß für den Fall der Glucosebestimmung in Gegenwart anderer optisch aktiver Blutbestandteile. Durch die Strahlteilung wird ein vom Absolutwert des Signals unabhängig Analysenwert erhalten. Als Lichtquelle 1 kann sowohl eine herkömmliche Lichtquelle mit Polarisator als auch beispielsweise ein Laser oder eine Laserdiode verwendet werden. Das λ/4-Plättchen 4 dient zur Korrektur der Depolarisationswirkung beispielsweise von durchstrahlter Haut oder in der Nähe der Meßstelle liegender Gewebeteile. Der Verstärker 15 entspricht dem herkömmlichen Endverstärker.

Fig. 2 entspricht prinzipiell der Anordnung von Fig. 1, wobei jedoch zusätzlich ein Modulator 2 vorgesehen ist, der vom Frequenzgenerator 11 angesteuert wird. Als Modulator können beispielsweise ein Faraday-Modulator, ein Lichtmodulator auf der Basis des Kerr-Effekts, ein Lichtamplitudenmodulator wie beispielsweise eine gepulste Laserdiode oder ein Phasenmodulator, also eine die Lichtweglänge verändernde optische Einrichtung, dienen. Der Generator 11, der die Modulationsfrequenz liefert, ist mit dem Verstärker 9 verbunden, der auf die Modulationsfrequenz des Frequenzgenerators 11 abgestimmt ist. Als Verstärker 9 kann beispielsweise ein selektiver Verstärker dienen, insbesondere ein Lock-in-Verstärker. Die Anordnung von Fig. 2, bei der der Lichtstrahl vor dem Eintritt in die Probe moduliert und nach der Signalverarbeitung im Differenzverstärker bzw Quotientenbildner 8 im Verstärker 9 wieder demoduliert wird, dient zur Erhöhung der Meßempfindlichkeit.

Fig. 3 entspricht im Prinzip der Vorrichtung von Fig. 1, wobei die Selektion des der zu bestimmenden Komponente zugeordneten Drehwertinkrements aufgrund der unterschiedlichen Zeit-Konzentrations-Charakteristik unter Verwendung eines Differenzierglieds 12, eines Signalteilers 13, beispielsweise eines Hochpasses bei der in-vivo-Glucosebestimmung, und eine Integrators 14 vorgenommen wird.

Durch die Selektion des der zu bestimmenden Komponente zugeordneten Drehwerts von den Drehwertinkrementen, die durch optisch aktive Begleitsubstanzen mit abweichendem Zeit-Konzentrations-Verhalten bedingt sind, wird entsprechend eine quantitative Bestimmung der Konzentration einer optisch aktiven Komponente in Gegenwart anderer optisch aktiver Komponenten ermöglicht, sofern das Zeit-Konzentrations-Verhalten der zu bestimmenden Komponente von dem der Begleitkomponenten hinreichend verschieden ist.

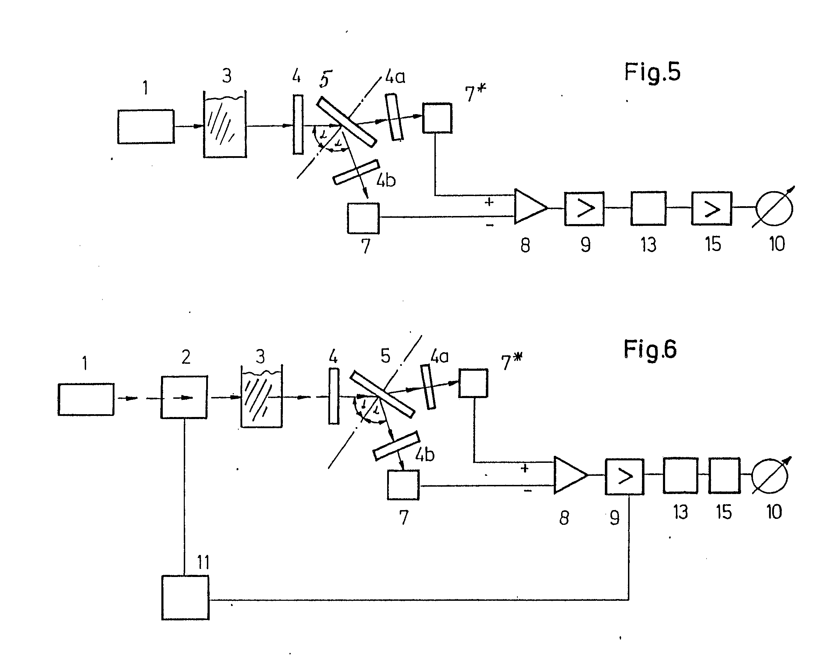

Das in Fig. 4 dargestellte herkömmliche Polariameter unterscheidet sich von der in Fig. 3 dargestellten durch den zusätzlichen Modulator 2, der vom Frequenzgenerator 11 gesteuert wird, sowie die Demodulationsstufe des Verstärkers 9, der auf die Modulationsfrequenz des Frequenzgenerators 11 abgestimmt ist. Durch diese Anordnung kann gegenüber dem Polarimeter von Fig. 3 eine Empfindlichkeitssteigerung um mindestens eine dekadische Größenordnung erzielt werden, wodurch entsprechend selektive quantitative Bestimmungen optisch aktiver Komponenten in sehr kleinen Konzentrationen möglich sind. Gleiches gilt für das Polarimeter von Fig. 2.

Das in Fig. dargestellte erfindungsmemäße Polarimeter beruht, abgesehen von der analogen Signalteilung unter Selektion des Drehwerts der zu bestimmenden Komponente wie in den Fig. 1, 2, 3 und 4, auf dem erfindungsgemäß neuartigen Konzept der Empfindlichkeitssteigerung durch Weglassen des Herkömmlichen Analysators, ggf bei gleichzeitiger Vertauschung von Meß- und Referenzzweig gegenüber herkömmlichen Anordnungen mit Strahlteiler und Analysator. Als Strahlteiler, der, wie oben theoretisch erläutert, auch als Analysator wirkt, ist in Fig. 5 eine planparallele Platte 5, die beispielsweise aus Kronglas besteht, verwendet. Die Polarisationsfilter 4a, 4b dienen zur Erzeugung der Parallel- bzw Senkrechtkomponente der Strahlungsintensität. Der an der Oberfläche reflektierte Strahl wird durch das Polarisationsfilter 4b zum Detektor 7 geleitet, der die Lichtintensität in ein elektrisches Signal umsetzt, während der gebrochene Anteil nach Durchlaufen der planparallelen Platte und des Polarisationsfilters 4a auf den Detektor 7* trifft und dort das Referenzsignal erzeugt. Im Differenzverstärker bzw Quotientenbildner 8 wird die Differenz bzw der Quotient der beiden Signale gebildet, was, nach der erläuterten Signalselektion der zu bestimmenden Komponente, in der Anzeige- oder Registrireinrichung 10 angezeigt bzw registriert wird. Durch geeignete Auswahl des Winkels α und ggf auch durch mindestens teilweise Verspiegelung der planparallelen Platte an der Rückseite oder Vorderseite kann die Intensität der auf die beiden Detektoren 7 und 7* gelangenden Strahlung auf den gleichen Wert eingestellt werden. Der Verstärker 15, der zur Korrektur einen angepaßten Phasengang (Allpaß) aufweisen kann, verstärkt dabei das Signal nochmals im Sinne eines Endverstärkers.

Das in Fig. 6 dargestellte erfindungsgemäße Polarimeter stellt eine besonders bevorzugte Ausführungsform dar und entspricht dem Polarimeter von Fig. 5 mit dem Unterschied, daß demgegenüber noch vom Modulationsprinzip Gebrauch gemacht ist, wobei das von der Lichtquelle emittierte Licht durch den vom Frequenzgenerator 11 gesteuerten Modulator 2 moduliert und anschließend im Demodulator 9 in abgestimmter Weise wieder demoduliert wird, wodurch die Empfindlichkeit ebenfalls gesteigert werden kann.

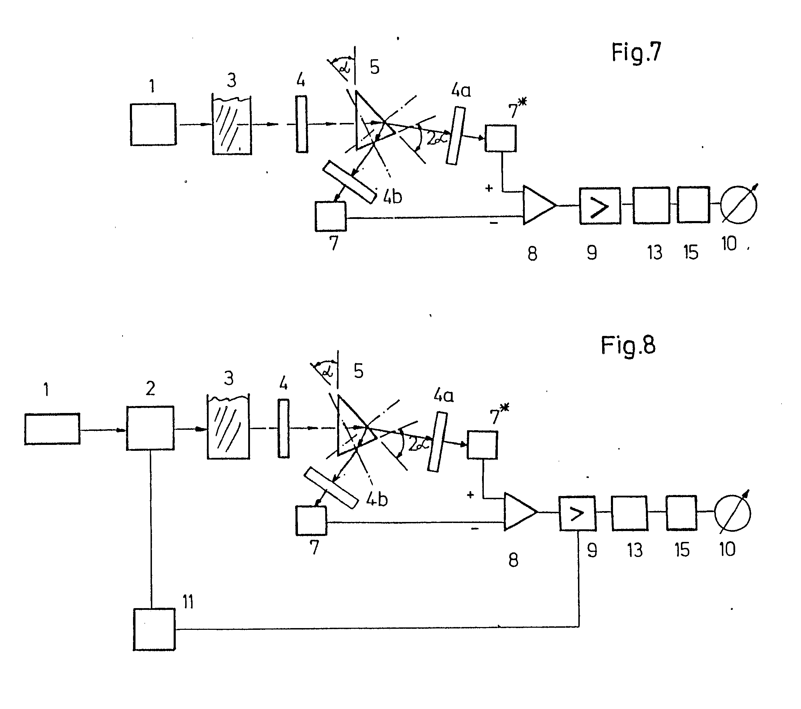

In Fig. 7 ist ein weiteres erfindungsgemäßes Polarimeter dargestellt, das als Strahlteiler mit Analysatorfunktion ein Prisma 5 aufweist. Die Reflexion erfolgt abweichend von den Polarimetern der Fig. 5 und 6, bei denen am optisch dichteren Medium reflektiert wird, am optisch dünneren Medium. Der polarisierte Lichtstrahl gelangt nach Durchlaufen der Probe 3 und des λ/4-Plättchens 4 zum Prisma 5, an dessen rückwärtiger Grenzfläche der Strahl unter dem Winkel α zur Flächennormalen auftrifft. Der Strahl wird dabei teilweise durchgelassen und teilweise reflektiert. Der reflektierte Teil des Strahls wird an der zweiten rückwärtigen Fläche nochmals reflektiert und teilweise durchgelassen. Beim Austritt aus dem Prisma wird der Strahl gebrochen, trifft nach dem Polarisationsfilter 4b auf den Detektor 7 und erzeugt die Signalspannung. Die durchgelassene Teilintensität der ersten Reflexion an der rückwärtigen Prismenfläche gelangt nach dem Polarisationsfilter 4a zum Detektor 7* und erzeugt dort ein Referenzsignal. Die Ausgangsspannungen der Detektoren 7 und 7* werden im Differenzverstärker bzw Quotientenbildner 8 subtrahiert bzw dividiert, im Verstärker 9 verstärkt, im Signalteiler 13 selektiert und nach Endverstärkung in der Anzeigeeinrichtung 10 angezeigt.

Das in Fig. 8 dargestellt Polarimeter stellt eine erfindungsgemäß besonders bevorzugte weitere Ausführungsform dar und entspricht bis auf die zusätzliche Modulation (Modulator 2, Frequenzgenerator 11, abgestimmter Verstärker 9) dem Polarimeter von Fig. 7.

Der Verstärker 15 ist zwar bei der Vornahme einer Frequenzbandtrennung theoretisch erforderlich, kann aber entfallen, wenn der Phasenfehler in der Eichkurve näherungsweise berücksichtigt wird.

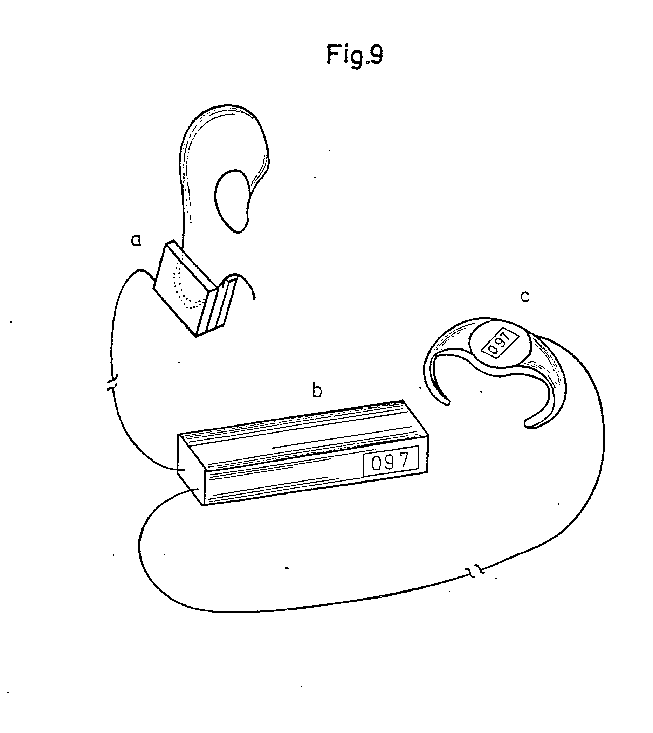

Der polarimetrische optische Teil des erfindungsgemäßen Polarimeters kann miniaturisiert bzw mikrominiaturisiert und vom Stromversorgungs- und Signalverarbeitungsteil bzw vom Anzeigeteil getrennt sein, was insbesondere für in-vivo-Messungen vorteilhaft ist. In Fig. 9 ist ein Ausführungsbeispiel für ein derartiges erfindungsgemäßes Polarimeter dargestellt, bei dem der optische bzw polarimetrische Meßteil a miniaturisiert und vom Stromversorgungs- und Signalverarbeitungsteil b getrennt ist und an einem geeigneten Körperteil wie dem Ohr bzw Ohrläppchen befestigt oder getragen werden oder auch in den Körper eingepflanzt sein kann. Der Stromversorgungs- und Signalverarbeitungsteil des Polarimeters kann zB in einer Jackentasche untergebracht werden. Die Anzeige erfolgt durch eine Anzeigeeinrichtung c, die beispielsweise nach Art einer Armbanduhr gestaltet ist und am Handgelenk getragen werden kann; die Anzeeigeinrichtung kann ferner auch in Schmuck wie beispielsweise einen Armreif, Ring odgl integriert sein.

Ferner kann der Meßteil a mit einem (zB druckenden) Kleinrechner und/oder einem Zeitgenerator gekoppelt werden, wobei eine automatische Aufzeichnung der Konzentrationswerte erreicht werden kann.

Bei allen Ausführungsformen, insbesondere jedoch beim implantierbaren Aufbau, ist eine direkte Steuerung oder Regelung der Insulinzufuhr durch die Glucosemeßvorrichtung eine besonders vorteilhafte Weiterbildung; die Erfindung erlaubt jedoch auch allgemein in Abhängigkeit von der ermittelten Konzentration der optisch aktiven Substanz eine gesteuerte Zufuhr oder Eindosierung beliebiger anderer Flüssigkeiten in den Körper.

Die Anzeige der Konzentration der zu bestimmenden optisch aktiven Substanz, beispielsweise der Blutgluclose, kann sowohl im Stromversorgungs- und Signalverarbeitungsteil als auch davon getrennt erfolgen und digital oder analog sein. Es ist ferner auch möglich, daß bei einer einstellbaren oberen und/oder unteren Signal- und damit Konzentrationsschwelle ein akustisches und/oder optisches Warnsignal ausgelöst wird. Ferner kann die Konzentration der zu bestimmenden oder überwachenden Substanz beispielsweise durch Tastendruck oder automatisch durch ein optisches oder akustisches Signal angezeigt werden. Auch Konzentrationsänderungen wie beispielsweise ein Steigen oder Fallen des Blutzuckers können durch entsprechende Modifizierung des erfindungsgemäßen Polarimeters angezeigt oder signalisiert werden.

Der Schutzbereich der Verfahrensansprüche erstreckt sich nicht auf Diagnostizierverfahren gemäß Artikel 52 (4) EPÜ.

## Patentansprüche

1. Verfahren zur quantitativen Bestimmung einer gelösten optisch aktiven Substanz
   in wäßrigen oder nichtwäßrigen Lösungen durch Polarimetrie
   unter Strahlteilung des Lichtstrahls nach der Probe mit einem Strahlteiler und Erzeugung eines Meßstrahls und eines Referenzstrahls und
   Bildung eines Differenz- oder Quotientensignals aus den mit entsprechenden Detektoren erfaßten Signalen des Meß- und Referenzstrahls,
   wobei das polarisierte Licht der Lichtquelle vor dem Durchlaufen der Probe ggf frequenz-, phasen- oder amplitudenmoduliert wird und das aus den Signalen des Meß- und Referenzstrahls gebildete Differenz- oder Quotientensignal in entsprechender Weise demoduliert wird,
   dadurch gekennzeichnet, daß zur gleichzeitigen Strahlteilung und Erzielung einer Analysatorwirkung der Lichtstrahl nach der Probe unter einem Einfallswinkel (α) auf eine optische Grenzfläche gerichtet wird, der zwischen dem Polarisationswinkel und dem Grenzwinkel der Totalreflexion liegt.

2. Verfahren nach Anspruch 1, dadurch gekennzeichnet, daß zur Strahlteilung und Erzielung einer Analysatorwirkung eine planparallele Platte verwendet wird.

3. Verfahren nach Anspruch 1, dadurch gekennzeichnet, daß zur Strahlteilung und Erzielung einer Analysatorwirkung ein Prisma verwendet und der an der Grenzfläche zum optische dünneren Medium reflektierte und an der zweiten Grenzfläche zum optisch dünneren Medium gebrochene Strahl als reflektierter Strahl herangezogen wird.

4. Verfahren nach Anspruch 3, dadurch gekennzeichnet, daß ein Prisma verwendet wird, das im Dreieckquerschnitt solche Winkel besitzt, daß das eingestrahlte Licht auf beide Grenzflächen zum dünneren Medium hin unter dem gleichen Winkel auftritt.

5. Verfahren nach Anspruch 3 oder 4, dadurch gekennzeichnet, daß ein Prisma verwendet wird, das im Dreieckquerschnitt die Winkel α, 2α und (180—3α) aufweist.

6. Hochempfindliches Polarimeter mit einer Lichtquelle (1) für polarisiertes Licht, einem Probenteil zur Aufnahme der Probe (3), einem Strahlteiler (5), je einem Detektor (7, 7*) für den Meß- sowie den Referenzstrahl, einem Differenzverstärker oder Quotientenbildner (8), einem Signalverstärker (15) und einer Anzeige-, Ausgabe- oder Registriereinrichung (10), dadurch gekennzeichnet, daß als Strahlteiler sowie als Analysator eine planparallele Platte (5) derart angeordnet ist, daß der zum Einfallslot auf der reflektierenden Fläche hin gemessene Einfallswinkel α zwischen dem Grenzwinkel der Totalreflexion und dem Polarisationswinkel liegt.

7. Hochempfindliches Polarimeter mit einer Lichtquelle (1) für polarisiertes Licht, einem Probenteil zur Aufnahme der Probe (3) einem Strahlteiler (5), je einem Detektor (7, 7*) für den Meß- sowie den Referenzstrahl, einem Differenzverstärker oder Quotientenbildner (8), einem Signalverstärker (15) und einer Anzeige-, Ausgabe- oder Registriereinrichtung (10), dadurch gekennzeichnet, daß als Strahlteiler sowie als Analysator ein Prisma (5) mit den Prismenwinkeln α, 2α und (180—3α) im Dreieckquerschnitt derart angeordnet ist, daß der zum Einfallslot auf der reflektierenden Fläche hin gemessene Einfallswinkel α gleich dem Prismenwinkel α ist, wobei α zwischen dem Grenzwinkel der Totalreflexion und dem Polarisationswinkel liegt.

8. Polarimeter nach Anspruch 6 oder 7, dadurch gekennzeichnet, daß der optische Teil miniaturisiert und zum Tragen am Ohr bzw zur Messung am Ohrläppchen ausgebildet ist.

9. Polarimeter nach einem der Ansprüche 6 bis 8, dadurch gekennzeichnet, daß es mit einer Regel- und/oder Dosiereinrichtung verbunden ist und diese direkt steuert, die in Abhängigkeit von der ermittelten Konzentration der optisch aktiven Substanz eine Flüssigkeit, insbesondere Insulin, in den Körper eindosiert.

## Revendications

1. Procédé pour la détermination quantitative d'une substance à activité optique, dissoute

— dan des solutions aqueuses ou non aqueuses, par polarimétrie

— avec division du faisceau lumineux en aval de l'échantillon par un diviseur de faisceau et production d'un faisceau de mesure et d'un faisceau de référence,

— et formation d'un signal différentiel ou de quotient à partir des signaux des faisceaux de mesure et de référence, déterminés par des détecteurs appropriés,

— la lumière polarisée de la source étant le cas échéant modulée en fréquence, phase ou amplitude avant le passage dans l'échantillon et le signal différentiel ou de quotient produit à partir des signaux des faisceaux de mesure et de référence étant démodulés de façon appropriée,

ledit procédé étant caractérisé en ce que pour obtenir simultanément une division du faisceau et une action d'analyseur, le faisceau lumineux en aval de l'échantillon est dirigé sous un angle d'incidence (α) sur une interface optique, située entre l'angle de polarisation et l'angle limite de réflexion totale.

2. Procédé selon revendication 1, caractérisé par l'emploi d'un lame plan-parallèle pour la divison du faisceau et la production d'une action d'analyseur.

3. Procédé selon revendication 1, caractérisé en ce que pour la division du faisceau et la production d'une action d'analyseur, un prisme est utilisé et le faisceau réfléchi par l'interface vers le milieu à plus faible densité optique, puis réfracté par le seconde interface vers le milieu à plus faible densité optique est utilisé comme faisceau réfléchi.

4. Procédé selon revendication 3, caractérisé par l'emploi d'un prisme qui, de section triangulaire, présente des angles tels que la lumière incidente atteint les deux interfaces avec le milieu de plus faible densité optique sous le même angle.

5. Procédé selon une des revendications 3 ou 4, caractérisé par l'emploi d'un prisme dont la section triangulaire présente les angles α, 2α et (180—3α).

6. Polarimètre de sensibilité élevée, comportant une source (1) de lumière polarisée, un support pour l'échantillon (3), un diviseur de faisceau (5), un détecteur (7, 7*) pour chacun des faisceaux de mesure et de référence, un amplificateur différentiel ou un logomètre (8), un amplificateur de signal (15) et un dispositif indicateur, de sortie ou enregistreur (10), ledit polarimétrie étant caractérisé en ce que le diviseur de faisceau et l'analyseur sont constitués par une lame plan-parallèle (5) disposée de façon que l'angle d'incidence α, mesuré par rapport à la normale à la surface réfléchissante, est compris entre l'angle limite de réflexion totale et l'angle de polarisation.

7. Polarimètre de sensibilité élevée, comportant une source (1) de lumière polarisée, un support pour l'échantillon (3), un diviseur de faisceau (5), un détecteur (7, 7*) pour chacun des faisceaux de mesure et de référence, un amplificateur différentiel et on logomètre (8), un amplificateur de signal (15) et un dispositif indicateur, de sortie ou enregistreur (10), ledit polarimètre étant caractérisé en ce que le diviseur de faisceau et l'analyseur sont constitués par un prisme (5) dont la section triangulaire présente les angles α, 2α et (180—3α) et disposé de façon que l'angle d'incidence α, mesuré par rapport à la normale à la surface réfléchissante, est égal à l'angle α du prisme, α étant compris entre l'angle limite de réflexion totale et l'angle de polarisation.

8. Polarimètre selon une des revendications 6 ou 7, caractérisé en ce que la partie optique est miniaturisée et réalisée pour être portée sur l'oreille ou pour mesure sur le lobe de l'oreille.

9. Polarimètre selon une quelconque des revendications 6 à 8, caractérisé en ce qu'il est relié à un dispositif régulateur et/ou doseur qu'il commande directement et qui dose dans le corps un liquide, et en particulier de l'insuline, en fonction de la concentration déterminée de la substance à activité optique.

**Claims**

1. A process for the quantitative determination of a dissolved optically active substance in aqueous or non-aqueous solutions by means of polarimetry by splitting the light beam after the sample by means of a beam splitter, thereby producing a measuring beam and a reference beam, and forming a difference or quotient signal of the signals of the measuring and the reference beam detected by corresponding detectors, whereby the polarized light of the light source prior to passing through of the sample is eventually subjected to frequency, phase, or amplitude modulation, and the difference or quotient signal formed of the signals of the measuring and the reference beam is demodulated correspondingly, characterized in that for simultaneously obtaining beam splitting and an analyzing effect the light beam after the sample is directed to an optical interface at an incidence angle (α) between the polarization angle and the limit angle of total reflection.

2. A process according to claim 1, characterized in that for obtaining beam splitting and an analyzing effect a plane parallel plate is used.

3. A process according to claim 1, characterized in that for obtaining beam splitting and an analyzing effect a prism is used and the beam reflected at the interface to the optically thinner medium and refracted at the second interface to the optically thinner medium is used as reflected beam.

4. A process according to claim 3, characterized in that a prism is used having such angles of the triangular cross section that the irradiated light is impacting on both interfaces in the direction of the thinner medium at the same angle.

5. A process according to claim 3 or 4, characterized in that a prism is used having the angles α, 2α, and (180—3α) of the triangular cross section.

6. A highly sensitive polarimeter comprising a light source (1) of polarized light, a sample part for taking-up a sample (3), a beam splitter (5), detectors (7, 7*) for the measuring and the reference beam, respectively, a differential amplifier or quotient former (8), a signal amplifier (15), and a display, output, or registering unit (10),

characterized in that as beam splitter and analyzer as well a plane parallel plate (5) is arranged in such a way that the angle α of incidence measured towards the perpendicular on the reflecting surface is between the limit angle of total reflection and the polarization angle.

7. A highly sensitive polarimeter comprising a light source (1) of polarized light a sample part for taking-up a sample (3), a beam splitter (5), detectors (7, 7*) for the measuring and the reference beam, respectively, a differential amplifier or quotient former (8), a signal amplifier (15), and a display, output, or registering unit (10), characterized in that as beam splitter and analyzer as well a prism (5) having the angles α, 2α, and (180—3α) of the triangle cross section is arranged in such a way that the angle α of incidence measured towards the perpendicular on the reflecting surface is equal to the angle α of the prism, α being between the limit angle of total reflection and the polarization angle.

8. A polarimeter according to claim 6 or 7, characterized in that the optical part is miniaturized and designed to be fastened to an ear and to carry out the measuring at an earlobe, respectively.

9. A polarimeter according to any one of claims 6 to 8, characterized in that it is connected to a controlling and/or a dosing unit and controls said unit directly, said unit dosing a liquid, particularly insulin, into the body in dependence of the detected concentration of the optically active substance.

Fig.1

Fig.2

Fig.3

Fig.4

Fig.5

Fig.6

Fig.7

Fig.8

# Fig.9